# EUROPEAN PATENT SPECIFICATION

(11) **EP 4 227 945 B1**
(45) Date of publication and mention of the grant of the patent: **18.09.2024**
(21) Application number: 23151692.3
(22) Date of filing: 16.01.2023
(51) Int. Cl.: G16B 15/30

(54) **METHOD AND DEVICE FOR PROTEIN-PROTEIN DOCKING BASED ON IDENTIFICATION OF LOW-ENTROPY HYDRATION LAYER ON PROTEIN SURFACE**
VERFAHREN UND VORRICHTUNG ZUR PROTEIN-PROTEIN-ANDOCKUNG AUF BASIS DER IDENTIFIZIERUNG EINER HYDRIERUNGSSCHICHT MIT GERINGER ENTROPIE AUF EINER PROTEINOBERFLÄCHE
PROCÉDÉ ET DISPOSITIF POUR L'ACCUEIL PROTÉINE-PROTÉINE SUR LA BASE DE L'IDENTIFICATION D'UNE COUCHE D'HYDRATATION À FAIBLE ENTROPIE SUR UNE SURFACE PROTÉIQUE

(30) Priority: 15.02.2022 CN 202210138581
(43) Date of publication of application: 16.08.2023
(73) Proprietor: SHENZHEN GUANZHAN BIOTECHNOLOGY CO., LTD., Shenzhen, Guangdong Province 518057 (CN)
(72) Inventor: YANG, Lin, Harbin - Heilongjiang, 150001 (CN); HOU, Chengyu, Harbin - Heilongjiang, 150001 (CN); LIAO, Chenchen, Harbin - Heilongjiang, 150001 (CN); HE, Xiaodong, Harbin - Heilongjiang, 150001 (CN)
(74) Representative: Porta & Consulenti Associati S.p.A.

(56) References cited:
- JIACHENG LI ET AL: "Hydrophobic interaction determines docking affinity of SARS CoV 2 variants with antibodies", ARXIV.ORG, CORNELL UNIVERSITY LIBRARY, 201 OLIN LIBRARY CORNELL UNIVERSITY ITHACA, NY 14853, 28 February 2021 (2021-02-28), XP081893375
- MOE SARAH J ET AL: "Mechanical Unfolding of Spectrin Repeats Induces Water-Molecule Ordering", BIOPHYSICAL JOURNAL, ELSEVIER, AMSTERDAM, NL, vol. 118, no. 5, 16 January 2020 (2020-01-16), pages 1076 - 1089, XP086083403, ISSN: 0006-3495, [retrieved on 20200116], DOI: 10.1016/J.BPJ.2020.01.005
- YANG LIN ET AL: "Spatial Layouts of Low-Entropy Hydration Shells Guide Protein Binding", GLOBAL CHALLENGES, 2 May 2023 (2023-05-02), XP093059380, ISSN: 2056-6646, DOI: 10.1002/gch2.202300022
- YANG LIN ET AL: "Low-Entropy Hydration Shells at the Spike RBD's Binding Site May Reveal the Contagiousness of SARS-CoV-2 Variants", BIOMOLECULES, vol. 13, no. 11, 7 November 2023 (2023-11-07), CH, pages 1628, XP093150751, ISSN: 2218-273X, Retrieved from the Internet <URL:https://www.ncbi.nlm.nih.gov/pmc/articles/PMC10669249/pdf/biomolecules-13-01628.pdf> DOI: 10.3390/biom13111628

## Description

### TECHNICAL FIELD

The present disclosure relates to a method and a device for predicting a protein-protein docking structure.

### BACKGROUND

Proteins and their products are the basis of life on the Earth and are involved in almost all known biochemical reactions and phenomena of life. There are trillions of different proteins in Earth organisms, with the biological function and activity of each protein expressed through a unique three-dimensional shape. A process by which proteins form natural three-dimensional structures is called protein folding, with its mechanism and laws as a basis of molecular biology, biophysics, and biochemistry. Protein folding is thought to be primarily guided by a variety of physical forces: (i) hydrogen bond formation, (ii) Van der Waals' forces, (iii) electrostatic forces, (iv) hydrophobic interactions, (v) entropy, and (vi) temperature. Protein folding realizes the functionalization of polypeptide chains, and interpretation and prediction of the protein folding is of great significance to biology, pathology, genetics, and pharmacology.

The tertiary structure of proteins is formed by the folding of polypeptide chains. A process of the tertiary structures being assembled into a quaternary structure as subunits is essentially the same as that of protein docking. In the quaternary structure, the subunits are mainly combined by hydrophobic interactions, followed by hydrogen bonds and an extremely small amount of ionic bonds. The hydrogen bonding between subunit structures generally occurs between the hydrophilic groups on a surface of the subunit. The formation of these hydrogen bonds also requires the hydrophilic side chains on a surface of the subunit structure to get rid of the hydrogen bonding of environmental water molecules. The hydrophilicity of a residue side chain is generally expressed only by C-O or N-H groups at a top of the side chain. According to enthalpy calculation, the hydrogen bonding between the C-O group and the N-H group on the side chain between the subunits may also lead to an increase in the enthalpy of a system. Therefore, hydrophilic groups on the surface of the subunit structure cannot spontaneously get rid of the hydrogen bonding of surface water molecules, such that the formation of hydrogen bonds between subunits also requires enthalpy-entropy compensation. For the tertiary structure as a subunit, its surface is generally distributed with localized hydrophobic regions. During the formation of a quaternary structure of proteins, a local hydrophobic collapse between subunits at a docking interface can provide a source of the enthalpy-entropy compensation and promote the formation of hydrogen bonds between subunits, thereby forming a precise quaternary structure. Therefore, the assembly of subunits into a quaternary structure is driven by hydrophobic interactions and enthalpy-entropy compensation mechanisms.

It must be pointed out that the protein surface has a hydration layer with a thickness of about 1 nm to 2 nm, and it is found that the dynamic behavior of water molecules in the hydration layer is significantly different from that of free water molecules. The experimental data published by Dongping Zhong in Proceedings of the National Academy of Sciences (PNAS) shows that the water molecules in the hydration layer on the protein surface have a movement speed of only one percent of that of the free water molecules, and the water molecules in the hydration layer has lower entropy. The long-range hydrophobic interactions between protein molecules and a resulting entropy increase should be a core driving force for protein-protein docking. Due to the existence of hydration layers, the enthalpy of a system may increase due to hydrogen bonding and electrostatic interactions between protein molecules, such that the protein-protein docking is driven by enthalpy-entropy compensation.

Jicheng et al ("Hydrophobic interaction determines docking affinity of SARS CoV 2 variants with antibodies",ARXIV.ORG, CORNELL UNIVERSITY LIBRARY, 201 OLIN LIBRARY CORNELL UNIVERSITY ITHACA, NY 14853, 28 February 2021) discloses the determination of hydrophobicity on the surface of proteins stabilizing protein-protein binding. Changes in amino acids on the surface increasing hydrophobic attraction and decreasing hydrophilic repulsion is investigated, in particular carbonyl vs amino groups. The results in MOE SARAH J ET AL ("Mechanical Unfolding of Spectrin Repeats Induces Water-Molecule Ordering",BIOPHYSICAL JOURNAL, ELSEVIER, AMSTERDAM, NL, vol. 118, no. 5, 16 January 2020 (2020-01-16), pages 1076-1089) indicate that upon mechanically induced unfolding of the protein, the solvent molecules become more ordered and increase their average number of hydrogen bonds. In addition, the unfolded structures originating from mechanical pulling expose an increasing amount of the hydrophobic residues to the solvent molecules, and the uncoiled regions adapt a convex surface with a small radius of curvature. As a result, the solvent molecules reorganize around the protein's small protrusions in structurally ordered waters that are characteristic of the so-called "small-molecule regime," which allows water to maintain a high hydrogen bond count at the expense of an increased structural order.

The mechanism of protein-protein interaction and recognition is an important topic in biology, pathology, genetics, and pharmacology. The sites of protein-protein interactions represent important viral infection and immune mechanisms. Taking the current global pandemic COVID-19 as an example, the root cause lies in the specific and high-affinity binding between a spike protein (S protein) of the COVID-19 virus and a human ACE2 protein. That is to say, the "protein-protein docking" is a core problem of researches on virus infection mechanism.

In an early stage of the epidemic, due to the lack of effective methods to predict a molecular structure and binding energy of a binding state of the virus and the receptor, experimental methods generally can only quickly decipher gene sequence information of the virus, while the gene sequence information cannot be used to directly decipher important information such as virus infectivity and probability of vaccine breakthrough infection. Therefore, it is an inevitable trend of technological development in this field to predict a complex structure of the protein-protein docking by computational methods. However, the currently popular computational methods for protein-protein docking assist in predicting the structure of protein complexes using a scoring function selected from natural conformation, while there are still relatively few studies on a physical mechanism of the protein-protein docking based on "enthalpy-entropy compensation" to solve this important scientific problem.

In view of the above research background, the present disclosure proposes a method for protein-protein docking based on identification of a low-entropy hydration layer on a protein surface. Different from traditional protein-protein docking prediction algorithms based on noncovalent interactions (such as electrostatic interactions, Van der Waals' forces, hydrogen bonds, and ionic bonds), the method can find a matching relationship of the hydrophobic interactions between proteins based on identifying a low-entropy region of the hydration layer on the protein surface, thereby accurately predicting a protein-protein docking structure. This novel method elucidates the essential drivers of protein recognition and docking, and resolves the principles of protein interactions, which is of great significance for understanding protein function and treating protein-related diseases. This method can provide a number of important virus infection mechanism information in the absence of clinical data, provide a scientific data support for the decision-making of accurate and effective national defense and epidemic prevention measures, and improve a rapid response capability of military and civilians to virus epidemic prevention and treatment. In addition, the method can be used to predict action sites of the proteins and drug molecules, and effectively evaluate and screen the interaction sites that are close to a real state. Therefore, the method can avoid blind, inefficient, and low-quality drug development processes, shorten a drug molecule development time, and save a lot of manpower and material resources.

### SUMMARY

In order to avoid inaccurate sites of current protein docking, the present disclosure proposes a new method for identifying a low-entropy region in a hydration layer on a protein surface. The hydration layers of certain hydrophilic groups surrounded by hydrophobic groups on the protein surface are identified as low-entropy regions, which are a result of surface tension and confinement. This new method for predicting a structure of a protein complex can rapidly and accurately predict a complex structure of protein-protein docking.

The present disclosure provides a method for protein-protein docking based on identification of a low-entropy hydration layer on a protein surface, including the following steps:
step 1, rubbing heavy atoms on a protein surface, where the heavy atoms include carbon atoms, nitrogen atoms, oxygen atoms, and sulfur atoms; calculating an average spatial coordinate of the heavy atoms in the protein, and dividing protein atoms into 20 regions with 20 vertices of a regular dodecahedron and using the average spatial coordinate as a center of projection;
step 2, for the protein in each obtained rubbing area, using a direction of the center of projection to the vertices of the regular dodecahedron as a z-axis, and determining surface atoms of the protein according to a maximum distance z from the center of projection to the protein atoms on the z-axis;
step 3, traversing the surface atoms, and changing a hydrophobicity and a hydrophilicity of the atoms according to amino acids and the surface atoms of the protein surface:
   case 1: if the amino acids of the protein surface are selected from the group consisting of leucine (Leu), tyrosine (Tyr), tryptophan (Trp), isoleucine (Ile), methionine (Met), phenylalanine (Phe), arginine (Arg), and lysine (Lys), and when carbonyl oxygen atoms or amide nitrogen atoms on backbone of the amino acids are exposed on the protein surface, changing the carbonyl oxygen atoms or the amide nitrogen atoms on the backbone to hydrophobic atoms, indicating that hydrophilic oxygen and nitrogen atoms of the backbone of residue Leu, Tyr, Trp, Ile, Met, Phe, Arg, or Lys become the hydrophobic atoms;
   case 2: if the amino acids of the protein surface belong to residue Trp, Tyr, Lys, and the Met, changing oxygen and nitrogen atoms of side chains of the amino acids to the hydrophobic atoms, indicating that the hydrophilic oxygen and nitrogen atoms at a head of the side chains of residue Trp, Tyr, Lys, and Met become the hydrophobic atoms; and
   case 3: if hydrogen bonds are formed between the oxygen and nitrogen atoms of the protein surface, whether in an "a-helix" and a "β-sheet" of a secondary structure of the protein or in elsewhere, changing the oxygen and nitrogen atoms that form the hydrogen bonds to the hydrophobic atoms;
step 4, after changing the hydrophobicity and the hydrophilicity of the atoms in the 20 regions of the protein in step 3, re-fitting a plane by a least square method to the surface atoms of the protein in each of the 20 regions, as a candidate protein docking plane; calculating an average distance di between a central coordinate position (xi, yi) of each of the protein docking planes in the 20 regions and all atoms on the plane, where i is a serial number of the rubbing area;
after fitting 20 planes, comparing obtained planes and excluding duplicate planes; during excluding the duplicate planes, if two fitting planes have an included angle of less than or equal to 10° from a spatial origin to vertical vectors of the respective planes, regarding the two fitting planes as a same plane; and
denoting remaining fitting planes as surface planes, and for each surface plane, selecting a central atom in a hydrophobic connection region, and marking atoms in the hydrophobic connection region;
step 5, calculating a hydrophobic area of the hydrophobic atoms on a surface of each hydrophobic connection region separately; and
step 6, selecting first three hydrophobic connection regions with a maximum hydrophobic area in the protein as possible docking positions, and docking two proteins to be docked. docked;
wherein step 1 specifically comprises: reading data information in a protein data bank (PDB) structure file of the protein, to obtain a three-dimensional spatial coordinate of each heavy atom on the protein surface; and dividing the protein atoms into the 20 regions with the 20 vertices of the regular dodecahedron as follows: the regular dodecahedron has the 20 vertices, and the average spatial coordinate of all atoms of the protein is used as a spatial origin; if in a spatial area, an angle is less than 41° between a vector pointing to each atom from the spatial origin and a vector pointing to the vertex, the atom is divided into this spatial area; the 20 vertices of the regular dodecahedron divide the protein atoms into 20 areas, and one divided area of the protein surface is regarded as one rubbing area; and wherein in step 2, a process of determining the surface atoms of the protein according to the maximum distance z from the center of projection to the protein atoms on the z-axis specifically comprises: selecting externally lateral atoms on 30% of the maximum distance z from the center of projection to the protein atoms on the z-axis as the surface atoms of the protein, that is, selecting atoms with a distance from the center of projection of greater than 70% × dsurface as the surface atoms of the protein, wherein the dsurface is a distance from the center of projection to a maximum z-coordinate of the atoms of the protein surface on the z-axis.

Further, step 1 includes the following steps:
reading data information in a protein data bank (PDB) structure file of the protein, to obtain a three-dimensional spatial coordinate of each heavy atom on the protein surface; and
dividing the protein atoms into the 20 regions with the 20 vertices of the regular dodecahedron as follows: the regular dodecahedron has the 20 vertices, and the average spatial coordinate of all atoms of the protein is used as a spatial origin; if in a spatial area, an angle is less than 41° between a vector pointing to each atom from the spatial origin and a vector pointing to the vertex, the atom is divided into this spatial area; the 20 vertices of the regular dodecahedron divide the protein atoms into 20 areas, and one divided area of the protein surface is regarded as one rubbing area.

Further, in step 2, a process of determining the surface atoms of the protein according to the maximum distance z from the center of projection to the protein atoms on the z-axis specifically includes:
selecting externally lateral atoms on 30% of the maximum distance z from the center of projection to the protein atoms on the z-axis as the surface atoms of the protein, that is, selecting atoms with a distance from the center of projection of greater than 70% × d_{surface} as the surface atoms of the protein, where the d_{surface} is a distance from the center of projection to a maximum z-coordinate of the atoms of the protein surface on the z-axis.

Further, in step 4, a process of denoting the remaining fitting planes as the surface planes, and for each surface plane, selecting the central atom in the hydrophobic connection region specifically includes:
transforming atomic coordinates into a three-dimensional space with the surface plane as an xy plane by coordinate transformation; taking xy coordinates of the atomic coordinates, if in a circle centered on oxygen and nitrogen atoms and with a radius of 5 angstroms, there are no other oxygen and nitrogen atoms, not using this atom as a boundary in a subsequent search for the boundary; otherwise, in the xy plane, with oxygen and nitrogen atoms as the boundaries, assigning carbon and sulfur atoms as 1 in a circle centered on corresponding oxygen and nitrogen atoms and with a radius of 3 angstroms; taking assigned carbon and sulfur atoms as a center, if there are unassigned carbon and sulfur atoms in a circle centered on the assigned carbon and sulfur atoms and with a radius of 3 angstroms, adding up values of the assigned atoms in a circle centered on the unassigned carbon and sulfur atoms and with a radius of 3 angstroms as values of the unassigned carbon and sulfur atoms; at this time, only calculating the values of the unassigned carbon and sulfur atoms, and recording as pseudo-valuation of the unassigned carbon and sulfur atoms, while not assigning the unassigned carbon and sulfur atoms; when all atoms are searched for one round, assigning the pseudo-valuation of the unassigned carbon and sulfur atoms to the corresponding unassigned carbon and sulfur atoms, and starting a new round of assignment until all carbon and sulfur atoms complete the assignment; and
for each carbon and sulfur atom, in a circle centered on the carbon and sulfur atoms and with a radius of 3 angstroms, if values of the carbon and sulfur atoms are greater than or equal to values of surrounding atoms, regarding the carbon and sulfur atoms as central atoms of the corresponding hydrophobic connection region.

Further, in step 4, a process of marking atoms in the hydrophobic connection region specifically includes:
taking the central atom of the hydrophobic connection region as a center and 10° as a step size, dividing an area around the central atom, and selecting atoms with a value of greater than or equal to 3 in a fan-shaped area corresponding to each 10°; when an atom with a value of less than 3 appears for the first time, selecting a distance from an atom with a value of 3 closest to the atom with a value of less than 3 to the center as a cut-off distance; and selecting atoms within the cut-off distance as atoms within the hydrophobic connection region.

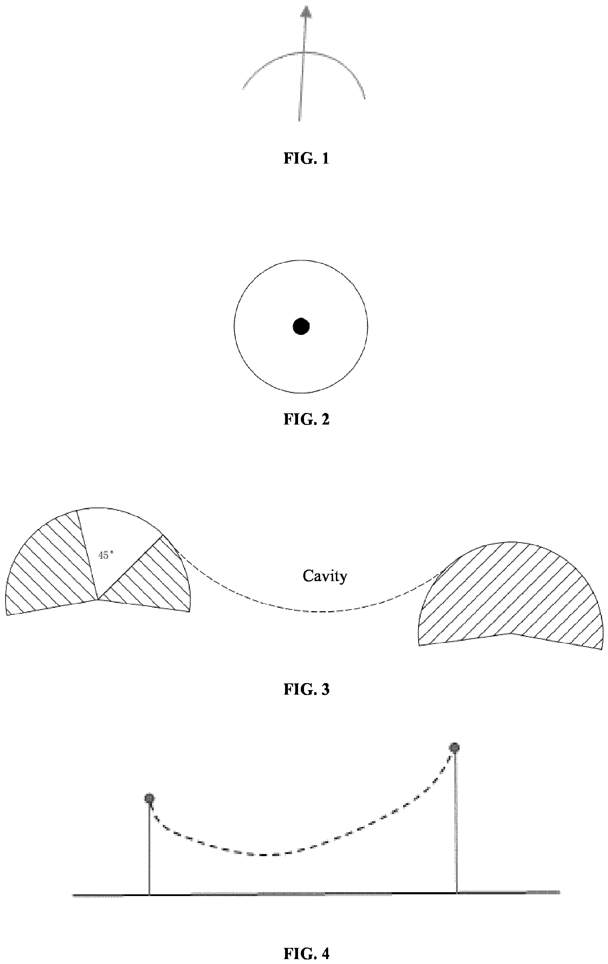

Further, in step 5, a process of calculating the hydrophobic area of the hydrophobic atoms on the surface of each hydrophobic connection region separately specifically includes:
step 5.1, for the surface heavy atoms of the protein in each hydrophobic connection region, displaying the surface heavy atoms as a sphere with an action radius of 1.8 angstroms; with each surface heavy atom as a center, making a hemisphere in a projection direction, where the hemisphere is a hemispherical shell;
step 5.2, establishing a two-dimensional grid with an interval of 0.1 angstrom on a plane of the hydrophobic connection region, and recording height information and a heavy atom type of the corresponding protein surface in each two-dimensional grid; and
step 5.3, establishing a surface with a radius of 1.8 angstroms as an action radius of the heavy atoms, with possibility of voids and discontinuities on the surface, where the hemispherical shells of the two heavy atoms have no intersection, and a distance between the two atoms is less than 6 angstroms, as shown in FIG. 3; taking a connection between a fan-shaped region formed by the hemispherical shell of one heavy atom with a projection direction in an included angle of 45° and a fan-shaped region formed by the hemispherical shell of another heavy atom with the projection direction in an included angle of 45° as an interpolation area; taking intersections of the connection and the two fan-shaped regions formed by the hemispherical shells of the two heavy atoms with the projection direction in an included angle of 45° as interpolation endpoints, obtaining a plane of a cavity between the hemispherical shells of the two heavy atoms by an interpolation, and abbreviating the plane of the cavity as an interpolation plane, as shown in FIG. 4; and
at the cavity, selecting a surface of the heavy atom at 45° to the projection direction as a surface connection point, and conducting cubic spline interpolation to obtain a type of the heavy atom and a three-dimensional height of the interpolation at the cavity, and then determining a space area corresponding to each grid in the two-dimensional grid, and then determining an area of the hydrophobic connection region.

Further, in step 6, a process of selecting the first three hydrophobic connection regions with a maximum hydrophobic area in the protein as the possible docking positions, and docking the two proteins to be docked specifically includes:
denoting the hydrophobic connection regions of the two proteins to be docked as a hydrophobic connection region A and a hydrophobic connection region B, respectively; determining a search range on the hydrophobic connection region A or the hydrophobic connection region B according to a relatively high average distance d=max(di, dj) corresponding to the two hydrophobic connection regions;
fixing the hydrophobic connection region A, establishing a 2d × 2d two-dimensional grid with an interval of 3 angstroms using a center coordinate of the hydrophobic connection region A as a surface origin of the hydrophobic connection region, and denoting area boundaries of the search range corresponding to the 2d × 2d of the hydrophobic connection region A as (x_max, y max); locating a center coordinate of the hydrophobic connection region B in grid points of the two-dimensional grid in sequence, and rotating at an interval of 5° and calculating a docking situation of the two hydrophobic connection regions at each grid point; a calculation method includes the following steps:
taking a normal vector of a fitting plane of each of the hydrophobic connection region A and the hydrophobic connection region B as a z-axis of the fitting plane, making the two hydrophobic connection regions approach on the z-axis of the fitting plane, placing the hydrophobic connection region B above the hydrophobic connection region A, and gradually reducing a height of the hydrophobic connection region B, that is, making the hydrophobic connection region B gradually approach the hydrophobic connection region A; where making the two hydrophobic connection regions gradually approach on the plane is to gradually overlap the z-axes of the two hydrophobic connection regions; however, if the z-axis of the hydrophobic connection region A faces upwards, the z-axis of the hydrophobic connection region B faces downwards, which is similar to conducting a coordinate transformation on the hydrophobic connection region B; and after the transformation, the two areas are in a same coordinate system;
according to the process of the hydrophobic connection region B gradually approaching the hydrophobic connection region A, setting a minimum distance to be 1 angstrom between the atoms on the hydrophobic connection region A and the hydrophobic connection region B, and denoting a position of the minimum distance as a space docking position; in the space docking position, finding out minimum x- and y-axis coordinates of the atomic coordinates of the hydrophobic connection region A and the hydrophobic connection region B, denoting as (x min, y_min), so as to facilitate the subsequent grid establishment and docking calculation;
after the coordinate (x_min, y_min) are obtained, pressing the atoms in the hydrophobic connection regions A and B to an x-y plane, calculating atoms closest to the coordinate (x min, y_min) in the hydrophobic connection regions A and B in the x-y plane, respectively, denoting two obtained atoms as an atom a and an atom b, taking an average of z-axis heights corresponding to respective spatial coordinates represented by the atom a and the atom b under the space docking position as a docking plane z value, and then determining a three-dimensional spatial coordinate (x_min, y_min, z); calculating a real distance from the atom a and the atom b in the space docking position separately using the coordinate (x min, y_min, z), and if there is a distance of not less than 6 angstroms in the two distances, determining that the hydrophobic connection region A and the hydrophobic connection region B have no docking surface at the coordinate; otherwise, determining that there is a docking surface between two proteins at the coordinate;
if there is no docking surface at the current space docking position, moving the hydrophobic connection region B on the two-dimensional grid of the hydrophobic connection region A, with a movement step size of (x, y) by 0.1 angstrom, that is: y increases by 0.1 once, and x goes from x_min to x_max; and y increases by 0.1 once more, and x goes from x_min to x_max once more; repeating the above step, with a range of y changing from y_min to y_max, until all coordinates are traversed;
when there is a docking surface between the two proteins, recording a type of the docking surface under the space docking position;
when there is a docking surface between the two proteins, determining a docking type at a docking interface of the hydrophobic connection regions A and B under the current space docking position, and adjusting the docking plane z value to a z-axis height corresponding to a same distance from the atom a and the atom b; where
the docking type includes carbon atom-carbon atom, carbon atom-oxygen and nitrogen atoms, and oxygen and nitrogen atoms-oxygen and nitrogen atoms, and areas of different docking types at the docking interface of the hydrophobic connection regions A and B are calculated according to the docking type.

Further, in step 6, if there are multiple docking positions, a maximum complete docking area is selected as the docking position, and a complete docking area of the two proteins is integrally calculated at the position.

The present disclosure further provides a device for protein-protein docking based on identification of a low-entropy hydration layer on a protein surface, including a memory, where the memory stores at least one instruction, and the at least one instruction is loaded and executed by a processor to implement the method for protein-protein docking based on identification of a low-entropy hydration layer on a protein surface.

Further, the device further includes a processor, where the processor loads and executes the at least one instruction stored in the memory to implement the method for protein-protein docking based on identification of a low-entropy hydration layer on a protein surface.

### Beneficial effects:

In the present disclosure, protein docking sites are predicted based on a low-entropy hydration layer mechanism. Area maximization and shape matching of low-entropy regions at the protein docking sites are the most effective means to determine protein-protein interaction sites, and are the latest achievements in current protein docking theory. The low-entropy hydration layer mechanism can accurately predict an interface and sites of the protein-protein docking, which is a brand-new protein-protein docking theory, and can rapidly and accurately predict a protein-protein docking structure. In addition, the method can be used to predict action sites of the proteins and drug molecules, and effectively evaluate and screen the action sites that are close to a real state.

### BRIEF DESCRIPTION OF THE DRAWINGS

FIG. 1 shows a schematic diagram of a hemispherical shell projection direction (front view) of an atomic surface;
FIG. 2 shows a schematic diagram of a connection point on a hemispherical shell in an included angle of 45° with a projection direction in a three-dimensional space (top view);
FIG. 3 shows a schematic diagram of an interpolation between surfaces of two unconnected atoms;
FIG. 4 shows a schematic diagram of height information of an interpolation plane; and
FIG. 5 shows an effect of a low-entropy hydration layer at docking sites of a protein surface in the present disclosure.

### DETAILED DESCRIPTION OF THE EMBODIMENTS

The present disclosure provides a method for protein-protein docking based on identification of a low-entropy hydration layer on a protein surface, and belongs to a novel method for predicting docking sites of the protein. The method for protein-protein docking based on identification of a low-entropy hydration layer on a protein surface follows these guidelines:
1, if the amino acids of the protein surface are selected from the group consisting of leucine (Leu), tyrosine (Tyr), tryptophan (Trp), isoleucine (Ile), methionine (Met), phenylalanine (Phe), arginine (Arg), and lysine (Lys), and when carbonyl oxygen atoms or amide nitrogen atoms on backbone of the amino acids are exposed on the protein surface, changing the carbonyl oxygen atoms or the amide nitrogen atoms on the backbone to hydrophobic atoms, indicating that hydrophilic oxygen and nitrogen atoms of the backbone of residue Leu, Tyr, Trp, Ile, Met, Phe, Arg, or Lys become the hydrophobic atoms;
2, if the amino acids of the protein surface belong to residue Trp, Tyr, Lys, and Met, changing oxygen and nitrogen atoms of residue side chains of the amino acids to the hydrophobic carbon atoms, indicating that the hydrophilic oxygen and nitrogen atoms at a head of side chains of residue Trp, Tyr, Lys, and Met become the hydrophobic atoms; and
3, if hydrogen bonds are formed between the oxygen and nitrogen atoms of the protein surface, whether in an "a-helix" and a "β-sheet" of a secondary structure of the protein or in elsewhere, changing the oxygen and nitrogen atoms that form the hydrogen bonds to the hydrophobic atoms.

Without displaying hydrogen atoms, distribution of the low-entropy hydration layer region on the protein surface is obtained by the above principles, and an area and a shape of the low-entropy hydration layer region are determined by a computer program, so as to obtain a low-entropy hydration layer sample with a larger area. By comparing the area and shape matching of the low-entropy hydration layers of two protein samples, these samples are compared overlappingly, and two samples are obtained whose graphic contours of the low-entropy hydration layer are closest to the two protein samples using a search algorithm. By docking the two proteins according to obtained contour lines, it is verified that whether space contours of a docking surface of the two proteins match. If the space contours match, a docking position model of the proteins can be obtained.

The following describes the present disclosure in detail with reference to specific implementations.

### Specific Implementation 1

The present implementation provides a method for protein-protein docking based on identification of a low-entropy hydration layer on a protein surface, including the following steps:
step 1, rubbing heavy atoms on a protein surface, where the heavy atoms include carbon atoms, nitrogen atoms, oxygen atoms, and sulfur atoms; calculating an average spatial coordinate of the heavy atoms in the protein, and decomposing the protein into 20 pieces in a direction of a rubbing area using the average spatial coordinate as a center of projection;
reading data information in a protein data bank (PDB) structure file of the protein, to obtain a three-dimensional spatial coordinate of each heavy atom on the protein; and
dividing the protein atoms into the 20 regions with the 20 vertices of the regular dodecahedron as follows: the regular dodecahedron has the 20 vertices, and the average spatial coordinate of all atoms of the protein is used as a spatial origin, namely the center of projection; if in a spatial area, an angle (an included angle between two vertex vectors) is less than 41° between a vector pointing to each atom from the spatial origin and a vector pointing to the vertex, the atom is divided into this spatial area; the 20 vertices of the regular dodecahedron divide the protein atoms into 20 areas, and one divided area of the protein surface is regarded as one rubbing area;
step 2, for a protein in each rubbing area, conducting projection from the projection center to the rubbing area; taking a region of the projection center to vertices of the regular dodecahedron as a z-axis, conducting projection on z-coordinates of all the atoms of the protein in the region onto the z-axis; selecting externally lateral atoms on 30% of the maximum distance z from the center of projection to the protein atoms on the z-axis as the surface atoms of the protein, that is, selecting atoms with a distance from the center of projection of greater than 70% × d_{surface} as the surface atoms of the protein, where the d_{surface} is a distance from the center of projection to a maximum z-coordinate of the atoms of the protein surface on the z-axis; where for different proteins, a distance from the center of projection to the vertex is different, and the program can automatically calculate the distance according to actual situations;
step 3, traversing the surface atoms, and changing a hydrophobicity and a hydrophilicity of the atoms according to amino acids and the surface atoms of the protein surface:
   case 1: if the amino acids of the protein surface are selected from the group consisting of leucine (Leu), tyrosine (Tyr), tryptophan (Trp), isoleucine (Ile), methionine (Met), phenylalanine (Phe), arginine (Arg), and lysine (Lys), and when carbonyl oxygen atoms or amide nitrogen atoms on backbone of the amino acids are exposed on the protein surface, changing the carbonyl oxygen atoms or the amide nitrogen atoms on the backbone to hydrophobic atoms, indicating that hydrophilic oxygen and nitrogen atoms of the backbone of the Leu, the Tyr, the Trp, the Ile, the Met, the Phe, the Arg, or the Lys become the hydrophobic atoms;
   case 2: if the amino acids of the protein surface belong to the Trp, the Tyr, the Lys, and the Met, changing oxygen and nitrogen atoms of side chains of the amino acids to the hydrophobic atoms, indicating that the hydrophilic oxygen and nitrogen atoms at a head of the side chains of the Trp, the Tyr, the Lys, and the Met become the hydrophobic atoms; and
   case 3: if hydrogen bonds are formed between the oxygen and nitrogen atoms of the protein surface, whether in an "a-helix" and a "β-sheet" of a secondary structure of the protein or in elsewhere, changing the oxygen and nitrogen atoms that form the hydrogen bonds to the hydrophobic atoms;
step 4, after changing the hydrophobicity and the hydrophilicity of the atoms in the 20 regions of the protein in step 3, re-fitting a plane by a least square method to the surface atoms of the protein in each of the 20 regions, as a candidate protein docking plane; calculating an average distance di between a central coordinate position (xi, yi) of each of the protein docking planes in the 20 regions and all atoms on the plane, where i is a serial number of the rubbing area;
after fitting 20 planes, comparing obtained planes and excluding duplicate planes; during excluding the duplicate planes, if two fitting planes have an included angle of less than or equal to 10° from a spatial origin to vertical vectors of the respective planes, regarding the two fitting planes as a same plane; and
denoting remaining fitting planes as surface planes, and for each surface plane, selecting a central atom in a hydrophobic connection region, and marking atoms in the hydrophobic connection region; where a method includes the following steps:
transforming atomic coordinates into a three-dimensional space with the surface plane as an xy plane by coordinate transformation; taking xy coordinates of the atomic coordinates (equivalent to compression to the xy plane), if in a circle centered on oxygen and nitrogen atoms and with a radius of 5 angstroms, there are no other oxygen and nitrogen atoms, not using this atom as a boundary in a subsequent search for the boundary; otherwise, in the xy plane (two-dimensional plane), with oxygen and nitrogen atoms as the boundaries, assigning carbon and sulfur atoms as 1 in a circle centered on corresponding oxygen and nitrogen atoms and with a radius of 3 angstroms; taking assigned carbon and sulfur atoms as a center, if there are unassigned carbon and sulfur atoms in a circle centered on the assigned carbon and sulfur atoms and with a radius of 3 angstroms, adding up values of the assigned atoms in a circle centered on the unassigned carbon and sulfur atoms and with a radius of 3 angstroms as values of the unassigned carbon and sulfur atoms; at this time, only calculating the values of the unassigned carbon and sulfur atoms, and recording as pseudo-valuation of the unassigned carbon and sulfur atoms, while not assigning the unassigned carbon and sulfur atoms; when all atoms are searched for one round, assigning the pseudo-valuation of the unassigned carbon and sulfur atoms to the corresponding unassigned carbon and sulfur atoms, and starting a new round of assignment until all carbon and sulfur atoms complete the assignment; and
for each carbon and sulfur atom, in a circle centered on the carbon and sulfur atoms and with a radius of 3 angstroms, if values of the carbon and sulfur atoms are greater than or equal to values of surrounding atoms, regarding the carbon and sulfur atoms as central atoms of the corresponding hydrophobic connection region;
taking the central atom of the hydrophobic connection region as a center and 10° as a step size, dividing an area around the central atom, and selecting atoms with a value of greater than or equal to 3 in a fan-shaped area corresponding to each 10°; when an atom with a value of less than 3 appears for the first time (since the central atom is used as a center of the divided area, values of the atoms decrease progressively in a direction from the center to an opening of each fan-shaped area), selecting a distance from an atom with a value of 3 closest to the atom with a value of less than 3 to the center as a cut-off distance; and selecting atoms within the cut-off distance as atoms within the hydrophobic connection region;
step 5, calculating a hydrophobic area of the hydrophobic atoms on a surface of each hydrophobic connection region separately; a method includes the following steps:
   step 5.1, for the surface heavy atoms of the protein in each hydrophobic connection region, displaying the surface heavy atoms as a sphere with an action radius of 1.8 angstroms; with each surface heavy atom as a center, making a hemisphere in a projection direction, where the hemisphere is a hemispherical shell, as shown in FIG. 1 and FIG. 2;
   step 5.2, establishing a two-dimensional grid with an interval of 0.1 angstrom on a plane of the hydrophobic connection region, and recording height information and a heavy atom type of the corresponding protein surface in each two-dimensional grid; and
   step 5.3, establishing a surface with a radius of 1.8 angstroms as an action radius of the heavy atoms, with possibility of voids and discontinuities on the surface, where the hemispherical shells of the two heavy atoms have no intersection, and a distance between the two atoms is less than 6 angstroms, as shown in FIG. 3; taking a connection between a fan-shaped region formed by the hemispherical shell of one heavy atom with a projection direction in an included angle of 45° and a fan-shaped region formed by the hemispherical shell of another heavy atom with the projection direction in an included angle of 45° as an interpolation area; taking intersections of the connection and the two fan-shaped regions formed by the hemispherical shells of the two heavy atoms with the projection direction in an included angle of 45° as interpolation endpoints, obtaining a plane of a cavity between the hemispherical shells of the two heavy atoms by an interpolation, and abbreviating the plane of the cavity as an interpolation plane, as shown in FIG. 4; and
at the cavity, selecting a surface of the heavy atom at 45° to the projection direction as a surface connection point, and conducting cubic spline interpolation to obtain a type of the heavy atom and a three-dimensional height of the interpolation at the cavity, and then determining a space area corresponding to each grid in the two-dimensional grid, and then determining an area of the hydrophobic connection region (space area); where
if the heavy atoms on both sides of the interpolation plane have a same type, the hydrophobic/hydrophilic types of the interpolation plane are the same as those on both sides; and if the heavy atoms on both sides of the interpolation plane have different types, using the middle as a boundary, the hydrophobic/hydrophilic types are different on both sides;
step 6, selecting first three hydrophobic connection regions with a maximum hydrophobic area in the protein as possible docking positions, and docking two proteins to be docked, as follows:
   denoting the hydrophobic connection regions of the two proteins to be docked as a hydrophobic connection region A and a hydrophobic connection region B, respectively; determining a search range on the hydrophobic connection region A or the hydrophobic connection region B according to a relatively high average distance d=max(di, dj) corresponding to the two hydrophobic connection regions;
   fixing the hydrophobic connection region A, establishing a 2d × 2d two-dimensional grid with an interval of 3 angstroms using a center coordinate of the hydrophobic connection region A as a surface origin of the hydrophobic connection region, and denoting area boundaries of the search range corresponding to the 2d × 2d of the hydrophobic connection region A as (x_max, y max); locating a center coordinate of the hydrophobic connection region B in grid points of the two-dimensional grid in sequence, and rotating at an interval of 5° and calculating a docking situation of the two hydrophobic connection regions at each grid point; a calculation method includes the following steps:
   taking a normal vector of a fitting plane of each of the hydrophobic connection region A and the hydrophobic connection region B as a z-axis of the fitting plane, making the two hydrophobic connection regions approach on the z-axis of the fitting plane, placing the hydrophobic connection region B above the hydrophobic connection region A, and gradually reducing a height of the hydrophobic connection region B, that is, making the hydrophobic connection region B gradually approach the hydrophobic connection region A; where making the two hydrophobic connection regions gradually approach on the plane is to gradually overlap the z-axes of the two hydrophobic connection regions; however, if the z-axis of the hydrophobic connection region A faces upwards, the z-axis of the hydrophobic connection region B faces downwards, which is similar to conducting a coordinate transformation on the hydrophobic connection region B; and after the transformation, the two areas are in a same coordinate system;
   according to the process of the hydrophobic connection region B gradually approaching the hydrophobic connection region A, setting a minimum distance to be 1 angstrom between the atoms on the hydrophobic connection region A and the hydrophobic connection region B, and denoting a position of the minimum distance as a space docking position; in the space docking position, finding out minimum x- and y-axis coordinates of the atomic coordinates of the hydrophobic connection region A and the hydrophobic connection region B, denoting as (x min, y_min), so as to facilitate the subsequent grid establishment and docking calculation;
   after the coordinate (x_min, y_min) are obtained, pressing the atoms in the hydrophobic connection regions A and B to an x-y plane, calculating atoms closest to the coordinate (x min, y_min) in the hydrophobic connection regions A and B in the x-y plane, respectively, denoting two obtained atoms as an atom a and an atom b, taking an average of z-axis heights corresponding to respective spatial coordinates represented by the atom a and the atom b under the space docking position as a docking plane z value, and then determining a three-dimensional spatial coordinate (x_min, y_min, z); calculating a real distance from the atom a and the atom b in the space docking position separately using the coordinate (x min, y_min, z), and if there is a distance of not less than 6 angstroms in the two distances, determining that the hydrophobic connection region A and the hydrophobic connection region B have no docking surface at the coordinate (only not at the current coordinate, but there is definitely a docking surface existing during the search); otherwise, determining that there is a docking surface between two proteins at the coordinate;
   if there is no docking surface at the current space docking position, moving the hydrophobic connection region B on the two-dimensional grid of the hydrophobic connection region A, with a movement step size of (x, y) by 0.1 angstrom, that is: y increases by 0.1 once, and x goes from x_min to x_max; and y increases by 0.1 once more, and x goes from x_min to x_max once more; repeating the above step, with a range of y changing from y_min to y_max, until all coordinates are traversed;
   when there is a docking surface between the two proteins, recording a type of the docking surface under the space docking position (carbon atom-carbon atom, carbon atom-oxygen and nitrogen atoms, and oxygen and nitrogen atoms-oxygen and nitrogen atoms);
   when there is a docking surface between the two proteins, determining a docking type at a docking interface of the hydrophobic connection regions A and B under the current space docking position, and adjusting the docking plane z value to a z-axis height corresponding to a same distance from the atom a and the atom b; where
   the docking type includes carbon atom-carbon atom, carbon atom-oxygen and nitrogen atoms, and oxygen and nitrogen atoms-oxygen and nitrogen atoms, and areas of different docking types at the docking interface of the hydrophobic connection regions A and B are calculated according to the docking type;
   if there are multiple docking positions, a maximum complete docking area is selected as the docking position, and a complete docking area of the two proteins is integrally calculated at the position.

### Example

In the present disclosure, based on related researches, a low-entropy hydration layer matching mechanism was proposed to identify protein docking sites. This mechanism revealed the mechanism of protein docking based on hydrophobic interaction. It was found by experiments that the hydrophobic interaction binding force between a COVID-19 virus S protein and an angiotensin converting enzyme ACE2 protein was much greater than that of a homologous SARS virus S protein and the ACE2 protein. The low-entropy hydration layer matching mechanism was consistent with results of an experiment published in "Science" and reasonably explained the experiment. This study showed that a reason why the COVID-19 virus was super infectious was that a maximum low-entropy hydration layer region on a surface of its S protein and a maximum low-entropy hydration layer region on a surface of its receptor protein had a high degree of matching, causing the COVID-19 virus to express an extraordinary receptor binding capacity, namely a super infectivity. Relevant studies had shown that the protein docking, including the COVID-19 virus, was dominated by hydrophobic forces, and hydrogen bonding and biochemical reactions between proteins were facilitated by mutual attraction of the maximum low-entropy hydration layers.

For protein-protein structure docking, an optimal binding site could be obtained by the distribution of low-entropy hydration layer regions on a surface of the protein structure. By analyzing three-dimensional images of the low-entropy hydration layer regions of the docking positions between hundreds of proteins, the images were compared with several maximum low-entropy hydration layer regions on the entire protein surface identified by a computer program. It was found that among several three-dimensional areas of the low-entropy hydration layers identified by the computer program, a projected area perfectly matching the low-entropy hydration layer could be found at an actual docking position. That is to say, hydrophobic docking sites between subunit structures were successfully predicted, which were fully in line with a theory that the maximum low-entropy hydration layer matching mechanism dominated the docking between protein structures to form protein-protein interactions. The results of program verification showed that the low-entropy hydration layer matching mechanism could accurately predict the docking position between protein-protein structures (referring to accompanying drawings in the description), demonstrating that the low-entropy hydration layer matching mechanism dominated by hydrophobic interactions drove the formation of protein-protein docking structures.

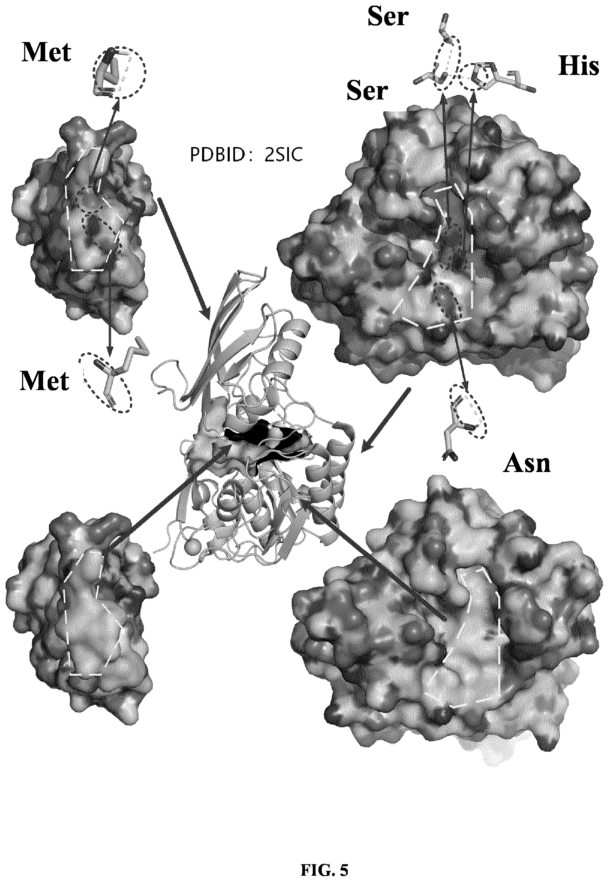

The method for protein-protein docking based on identification of a low-entropy hydration layer on a protein surface was described using the protein-protein docking of PDBID:2SIC as an example. The docking effect was shown in FIG. 5, where a middle figure represented the protein-protein docking, and a cyan part was a docking surface of the low-entropy hydration layer; upper two figures represented amino acids after PDBID:2SIC being manually manipulated to discolor according to discoloration criteria identified by the protein low-entropy hydration layer; and lower two figures showed that after treating by the discoloration criteria identified by the protein low-entropy hydration layer, the cyan part was a surface of the low-entropy hydration layer where the two proteins are docked.

### Specific Implementation 2

The present implementation provides a device for protein-protein docking based on identification of a low-entropy hydration layer on a protein surface, including a memory, further including a processor, where the memory stores at least one instruction, and the at least one instruction is loaded and executed by the processor to implement the method for protein-protein docking based on identification of a low-entropy hydration layer on a protein surface.

When the device includes only the memory, the device may be the memory itself.

## Claims

1. A method for protein-protein docking based on identification of a low-entropy hydration layer on a protein surface, comprising the following steps:
step 1, rubbing heavy atoms on a protein surface, wherein the heavy atoms comprise carbon atoms, nitrogen atoms, oxygen atoms, and sulfur atoms; calculating an average spatial coordinate of the heavy atoms in the protein, and dividing protein atoms into 20 regions with 20 vertices of a regular dodecahedron and using the average spatial coordinate as a center of projection;
step 2, for the protein in each obtained rubbing area, using a direction of the center of projection to the vertices of the regular dodecahedron as a z-axis, and determining surface atoms of the protein according to a maximum distance z from the center of projection to the protein atoms on the z-axis;
step 3, traversing the surface atoms, and identifying atoms as hydrophobic according to amino acids and the surface atoms of the protein surface, wherein
case 1: if the amino acids of the protein surface are selected from the group consisting of leucine (Leu), tyrosine (Tyr), tryptophan (Trp), isoleucine (Ile), methionine (Met), phenylalanine (Phe), arginine (Arg), and lysine (Lys), and when carbonyl oxygen atoms or amide nitrogen atoms on backbone of the amino acids are exposed on the protein surface, identifying the carbonyl oxygen atoms or the amide nitrogen atoms on the backbone as hydrophobic atoms, indicating that hydrophilic oxygen and nitrogen atoms of the backbone of residue Leu, Tyr, Trp, Ile, Met, Phe, Arg, or Lys become the hydrophobic atoms;
case 2: if the amino acids of the protein surface belong to residue Trp, Tyr, Lys, and Met, identifying oxygen and nitrogen atoms of residue side chains of the amino acids as the hydrophobic atoms, indicating that the hydrophilic oxygen and nitrogen atoms at a head of the side chains of residue Trp, Tyr, Lys, and Met become the hydrophobic atoms; and
case 3: if hydrogen bonds are formed between the oxygen and nitrogen atoms of the protein surface, whether in an "α-helix" and a "β-sheet" of a secondary structure of the protein or in elsewhere, identifying the oxygen and nitrogen atoms that form the hydrogen bonds as the hydrophobic atoms;
step 4, after identifying hydrophobic atoms in the 20 regions of the protein in step 3, re-fitting a plane, in each of the 20 regions, to the surface atoms of the protein by a least square method, each plane so obtained being used as a candidate protein docking plane; calculating an average distance di between a central coordinate position (xi, yi) of each of the protein docking planes in the 20 regions and all atoms on the plane, wherein i is a serial number of the rubbing area;
after fitting 20 planes, comparing obtained planes and excluding duplicate planes; during excluding the duplicate planes, if two fitting planes have an included angle of less than or equal to 10° from a spatial origin to vertical vectors of the respective planes, regarding the two fitting planes as a same plane; and
denoting remaining fitting planes as surface planes, and for each surface plane, selecting a central atom in a hydrophobic connection region, and marking atoms in the hydrophobic connection region;
step 5, calculating a hydrophobic area of the hydrophobic atoms on a surface of each hydrophobic connection region separately; and
step 6, selecting first three hydrophobic connection regions with a maximum hydrophobic area in the protein as possible docking positions, and docking two proteins to be docked;
wherein step 1 specifically comprises:
reading data information in a protein data bank (PDB) structure file of the protein, to obtain a three-dimensional spatial coordinate of each heavy atom on the protein surface; and
dividing the protein atoms into the 20 regions with the 20 vertices of the regular dodecahedron as follows: the regular dodecahedron has the 20 vertices, and the average spatial coordinate of all atoms of the protein is used as a spatial origin; if in a spatial area, an angle is less than 41° between a vector pointing to each atom from the spatial origin and a vector pointing to the vertex, the atom is divided into this spatial area; the 20 vertices of the regular dodecahedron divide the protein atoms into 20 areas, and one divided area of the protein surface is regarded as one rubbing area; and
wherein in step 2, a process of determining the surface atoms of the protein according to the maximum distance z from the center of projection to the protein atoms on the z-axis specifically comprises:
selecting externally lateral atoms on 30% of the maximum distance z from the center of projection to the protein atoms on the z-axis as the surface atoms of the protein, that is, selecting atoms with a distance from the center of projection of greater than 70% × d_{surface} as the surface atoms of the protein, wherein the d_{surface} is a distance from the center of projection to a maximum z-coordinate of the atoms of the protein surface on the z-axis.

2. The method for protein-protein docking based on identification of a low-entropy hydration layer on a protein surface according to claim 1, wherein in step 4, a process of denoting the remaining fitting planes as the surface planes, and for each surface plane, selecting the central atom in the hydrophobic connection region specifically comprises:
transforming atomic coordinates into a three-dimensional space with the surface plane as an xy plane by coordinate transformation; taking xy coordinates of the atomic coordinates, if in a circle centered on oxygen and nitrogen atoms and with a radius of 5 angstroms, there are no other oxygen and nitrogen atoms, not using this atom as a boundary in a subsequent search for the boundary; otherwise, in the xy plane, with oxygen and nitrogen atoms as the boundaries, assigning carbon and sulfur atoms as 1 in a circle centered on corresponding oxygen and nitrogen atoms and with a radius of 3 angstroms; taking assigned carbon and sulfur atoms as a center, if there are unassigned carbon and sulfur atoms in a circle centered on the assigned carbon and sulfur atoms and with a radius of 3 angstroms, adding up values of the assigned atoms in a circle centered on the unassigned carbon and sulfur atoms and with a radius of 3 angstroms as values of the unassigned carbon and sulfur atoms; at this time, only calculating the values of the unassigned carbon and sulfur atoms, and recording as pseudo-valuation of the unassigned carbon and sulfur atoms, while not assigning the unassigned carbon and sulfur atoms; when all atoms are searched for one round, assigning the pseudo-valuation of the unassigned carbon and sulfur atoms to the corresponding unassigned carbon and sulfur atoms, and starting a new round of assignment until all carbon and sulfur atoms complete the assignment; and
for each carbon and sulfur atom, in a circle centered on the carbon and sulfur atoms and with a radius of 3 angstroms, if values of the carbon and sulfur atoms are greater than or equal to values of surrounding atoms, regarding the carbon and sulfur atoms as central atoms of the corresponding hydrophobic connection region.

3. The method for protein-protein docking based on identification of a low-entropy hydration layer on a protein surface according to claim 2, wherein in step 4, a process of marking atoms in the hydrophobic connection region specifically comprises:
taking the central atom of the hydrophobic connection region as a center and 10° as a step size, dividing an area around the central atom, and selecting atoms with a value of greater than or equal to 3 in a fan-shaped area corresponding to each 10°; when an atom with a value of less than 3 appears for the first time, selecting a distance from an atom with a value of 3 closest to the atom with a value of less than 3 to the center as a cut-off distance; and selecting atoms within the cut-off distance as atoms within the hydrophobic connection region.

4. The method for protein-protein docking based on identification of a low-entropy hydration layer on a protein surface according to claim 3, wherein in step 5, a process of calculating the hydrophobic area of the hydrophobic atoms on the surface of each hydrophobic connection region separately specifically comprises:
step 5.1, for the surface heavy atoms of the protein in each hydrophobic connection region, displaying the surface heavy atoms as a sphere with an action radius of 1.8 angstroms; with each surface heavy atom as a center, making a hemisphere in a projection direction, wherein the hemisphere is a hemispherical shell;
step 5.2, establishing a two-dimensional grid with an interval of 0.1 angstrom on a plane of the hydrophobic connection region, and recording height information and a heavy atom type of the corresponding protein surface in each two-dimensional grid; and
step 5.3, establishing a surface with a radius of 1.8 angstroms as an action radius of the heavy atoms, with possibility of voids and discontinuities on the surface, wherein the hemispherical shells of the two heavy atoms have no intersection, and a distance between the two atoms is less than 6 angstroms, as shown in FIG. 3; taking a connection between a fan-shaped region formed by the hemispherical shell of one heavy atom with a projection direction in an included angle of 45° and a fan-shaped region formed by the hemispherical shell of another heavy atom with the projection direction in an included angle of 45° as an interpolation area; taking intersections of the connection and the two fan-shaped regions formed by the hemispherical shells of the two heavy atoms with the projection direction in an included angle of 45° as interpolation endpoints, obtaining a plane of a cavity between the hemispherical shells of the two heavy atoms by an interpolation, and abbreviating the plane of the cavity as an interpolation plane, as shown in FIG. 4; and
at the cavity, selecting a surface of the heavy atom at 45° to the projection direction as a surface connection point, and conducting cubic spline interpolation to obtain a type of the heavy atom and a three-dimensional height of the interpolation at the cavity, and then determining a space area corresponding to each grid in the two-dimensional grid, and then determining an area of the hydrophobic connection region.

5. The method for protein-protein docking based on identification of a low-entropy hydration layer on a protein surface according to claim 4, wherein in step 6, a process of selecting the first three hydrophobic connection regions with a maximum hydrophobic area in the protein as the possible docking positions, and docking the two proteins to be docked specifically comprises:
denoting the hydrophobic connection regions of the two proteins to be docked as a hydrophobic connection region A and a hydrophobic connection region B, respectively; determining a search range on the hydrophobic connection region A or the hydrophobic connection region B according to a relatively high average distance d=max(di, dj) corresponding to the two hydrophobic connection regions;
fixing the hydrophobic connection region A, establishing a 2d × 2d two-dimensional grid with an interval of 3 angstroms using a center coordinate of the hydrophobic connection region A as a surface origin of the hydrophobic connection region, and denoting area boundaries of the search range corresponding to the 2d × 2d of the hydrophobic connection region A as (x_max, y_max); locating a center coordinate of the hydrophobic connection region B in grid points of the two-dimensional grid in sequence, and rotating at an interval of 5° and calculating a docking situation of the two hydrophobic connection regions at each grid point; a calculation method comprises the following steps:
taking a normal vector of a fitting plane of each of the hydrophobic connection region A and the hydrophobic connection region B as a z-axis of the fitting plane, making the two hydrophobic connection regions approach on the z-axis of the fitting plane, placing the hydrophobic connection region B above the hydrophobic connection region A, and gradually reducing a height of the hydrophobic connection region B, that is, making the hydrophobic connection region B gradually approach the hydrophobic connection region A; wherein making the two hydrophobic connection regions gradually approach on the plane is to gradually overlap the z-axes of the two hydrophobic connection regions; however, if the z-axis of the hydrophobic connection region A faces upwards, the z-axis of the hydrophobic connection region B faces downwards, which is similar to conducting a coordinate transformation on the hydrophobic connection region B; and after the transformation, the two areas are in a same coordinate system;
according to the process of the hydrophobic connection region B gradually approaching the hydrophobic connection region A, setting a minimum distance to be 1 angstrom between the atoms on the hydrophobic connection region A and the hydrophobic connection region B, and denoting a position of the minimum distance as a space docking position; in the space docking position, finding out minimum x- and y-axis coordinates of the atomic coordinates of the hydrophobic connection region A and the hydrophobic connection region B, denoting as (x_min, y_min), so as to facilitate the subsequent grid establishment and docking calculation;
after the coordinate (x_min, y_min) are obtained, pressing the atoms in the hydrophobic connection regions A and B to an x-y plane, calculating atoms closest to the coordinate (x_min, y_min) in the hydrophobic connection regions A and B in the x-y plane, respectively, denoting two obtained atoms as an atom a and an atom b, taking an average of z-axis heights corresponding to respective spatial coordinates represented by the atom a and the atom b under the space docking position as a docking plane z value, and then determining a three-dimensional spatial coordinate (x_min, y_min, z); calculating a real distance from the atom a and the atom b in the space docking position separately using the coordinate (x_min, y_min, z), and if there is a distance of not less than 6 angstroms in the two distances, determining that the hydrophobic connection region A and the hydrophobic connection region B have no docking surface at the coordinate; otherwise, determining that there is a docking surface between two proteins at the coordinate;
if there is no docking surface at the current space docking position, moving the hydrophobic connection region B on the two-dimensional grid of the hydrophobic connection region A, with a movement step size of (x, y) by 0.1 angstrom, that is: y increases by 0.1 once, and x goes from x_min to x_max; and y increases by 0.1 once more, and x goes from x_min to x_max once more; repeating the above step, with a range of y changing from y_min to y_max, until all coordinates are traversed;
when there is a docking surface between the two proteins, recording a type of the docking surface under the space docking position;
when there is a docking surface between the two proteins, determining a docking type at a docking interface of the hydrophobic connection regions A and B under the current space docking position, and adjusting the docking plane z value to a z-axis height corresponding to a same distance from the atom a and the atom b; wherein
the docking type comprises carbon atom-carbon atom, carbon atom-oxygen and nitrogen atoms, and oxygen and nitrogen atoms-oxygen and nitrogen atoms, and areas of different docking types at the docking interface of the hydrophobic connection regions A and B are calculated according to the docking type.

6. The method for protein-protein docking based on identification of a low-entropy hydration layer on a protein surface according to claim 5, wherein in step 6, if there are multiple docking positions, a maximum complete docking area is selected as the docking position, and a complete docking area of the two proteins is integrally calculated at the position.

7. A device for protein-protein docking based on identification of a low-entropy hydration layer on a protein surface, comprising a memory, wherein the memory stores at least one instruction, and the at least one instruction is loaded and executed by a processor to implement the method for protein-protein docking based on identification of a low-entropy hydration layer on a protein surface according to any one of claims 1 to 6.

8. The device for protein-protein docking based on identification of a low-entropy hydration layer on a protein surface according to claim 7, further comprising a processor, wherein the processor loads and executes the at least one instruction stored in the memory to implement the method for protein-protein docking based on identification of a low-entropy hydration layer on a protein surface according to any one of claims 1 to 6.

## Patentansprüche

1. Verfahren zur Protein-Protein-Andockung auf Basis der Identifizierung einer Hydrierungsschicht mit geringer Entropie auf einer Proteinoberfläche, umfassend folgende Schritte:
Schritt 1, Reiben von Schweratomen auf einer Proteinoberfläche, wobei die Schweratome Kohlenstoffatome, Stickstoffatome, Sauerstoffatome und Schwefelatome umfassen; Berechnen einer durchschnittlichen räumlichen Koordinate der Schweratome in dem Protein und Unterteilen der Proteinatome in 20 Regionen mit 20 Scheitelpunkten eines regelmäßigen Dodekaeders und Verwenden der durchschnittlichen räumlichen Koordinate als Projektionszentrum;
Schritt 2, für das Protein in jedem erhaltenen Reibungsbereich, Verwenden einer Richtung des Projektionszentrums zu den Scheitelpunkten des regelmäßigen Dodekaeders als z-Achse und Bestimmen der Oberflächenatome des Proteins gemäß einem maximalen Abstand z vom Projektionszentrum zu den Proteinatomen auf der z-Achse;
Schritt 3, Durchlaufen der Oberflächenatome und Identifizieren der Atome als hydrophob gemäß den Aminosäuren und den Oberflächenatomen der Proteinoberfläche, wobei
Fall 1: wenn die Aminosäuren der Proteinoberfläche ausgewählt sind aus der Gruppe bestehend aus Leucin (Leu), Tyrosin (Tyr), Tryptophan (Trp), Isoleucin (Ile), Methionin (Met), Phenylalanin (Phe), Arginin (Arg) und Lysin (Lys), und wenn Carbonyl-Sauerstoffatome oder Amid-Stickstoffatome auf der Hauptkette der Aminosäuren auf der Proteinoberfläche exponiert sind, Identifizieren der Carbonyl-Sauerstoffatome oder der Amid-Stickstoffatome auf der Hauptkette als hydrophobe Atome, was anzeigt, dass die hydrophilen Sauerstoff- und Stickstoffatome der Hauptkette der Reste Leu, Tyr, Trp, Ile, Met, Phe, Arg oder Lys die hydrophoben Atome werden;
Fall 2: wenn die Aminosäuren der Proteinoberfläche zu den Resten Trp, Tyr, Lys und Met gehören, Identifizieren der Sauerstoff- und Stickstoffatome der Seitenketten der Aminosäuren als hydrophobe Atome, was anzeigt, dass die hydrophilen Sauerstoff- und Stickstoffatome am Kopf der Seitenketten der Reste Trp, Tyr, Lys und Met zu hydrophoben Atomen werden; und
Fall 3: wenn Wasserstoffbrücken zwischen den Sauerstoff- und Stickstoffatomen der Proteinoberfläche gebildet werden, sei es in einer "a-Helix" und einem "β-Faltblatt" einer Sekundärstruktur des Proteins oder an anderer Stelle, Identifizieren der Sauerstoff- und Stickstoffatome, die die Wasserstoffbrücken bilden, als hydrophobe Atome;
Schritt 4, nach dem Identifizieren hydrophober Atome in den 20 Regionen des Proteins in Schritt 3, erneutes Anpassen einer Ebene in jeder der 20 Regionen an die Oberflächenatome des Proteins durch eine Methode der kleinsten Quadrate, wobei jede so erhaltene Ebene als Kandidaten-Protein-Andockebene verwendet wird; Berechnen eines durchschnittlichen Abstands di zwischen einer zentralen Koordinatenposition (xi, yi) jeder der Protein-Andockebenen in den 20 Regionen und allen Atomen auf der Ebene, wobei i eine Seriennummer des Reibungsbereichs ist;
nach dem Anpassen von 20 Ebenen, Vergleichen der erhaltenen Ebenen und Ausschluss doppelter Ebenen; während des Ausschlusses der doppelten Ebenen, wenn zwei Anpassungsebenen einen eingeschlossenen Winkel von weniger als oder gleich 10° von einem räumlichen Ursprung zu vertikalen Vektoren der jeweiligen Ebenen aufweisen, Betrachten der zwei Anpassungsebenen als eine gleiche Ebene; und
Bezeichnen der verbleibenden Anpassungsebenen als Oberflächenebenen und für jede Oberflächenebene Auswählen eines Zentralatoms in einer hydrophoben Verbindungsregion und Markieren von Atomen in der hydrophoben Verbindungsregion;
Schritt 5: Berechnen eines hydrophoben Bereichs der hydrophoben Atome auf einer Oberfläche jeder hydrophoben Verbindungsregion getrennt; und
Schritt 6: Auswählen der ersten drei hydrophoben Verbindungsregionen mit einer maximalen hydrophoben Fläche im Protein als mögliche Andockpositionen und Andocken von zwei Proteinen, die angedockt werden sollen;
wobei Schritt 1 insbesondere umfasst:
Lesen von Dateninformationen in einer Proteindatenbank (PDB)-Strukturdatei des Proteins, um eine dreidimensionale räumliche Koordinate für jedes Schweratom auf der Proteinoberfläche zu erhalten; und
Aufteilen der Proteinatome in die 20 Regionen mit den 20 Scheitelpunkten des regelmäßigen Dodekaeders wie folgt: das regelmäßige Dodekaeder weist die 20 Scheitelpunkte auf, und die durchschnittliche räumliche Koordinate aller Atome des Proteins wird als räumlicher Ursprung verwendet; wenn in einem räumlichen Bereich ein Winkel von weniger als 41° zwischen einem Vektor, der auf jedes Atom vom räumlichen Ursprung zeigt, und einem Vektor, der auf den Scheitelpunkt zeigt, besteht, wird das Atom in diesen räumlichen Bereich unterteilt; die 20 Scheitelpunkte des regelmäßigen Dodekaeders unterteilen die Proteinatome in 20 Bereiche, und ein unterteilter Bereich der Proteinoberfläche wird als ein Reibungsbereich betrachtet; und
wobei in Schritt 2 ein Verfahren zur Bestimmung der Oberflächenatome des Proteins gemäß dem maximalen Abstand z vom Projektionszentrum zu den Proteinatomen auf der z-Achse insbesondere umfasst:
Auswählen von extern lateralen Atomen auf 30 % des maximalen Abstands z vom Projektionszentrum zu den Proteinatomen auf der z-Achse als die Oberflächenatome des Proteins, d. h. Auswählen von Atomen mit einem Abstand vom Projektionszentrum von mehr als 70 % x C_{Oberfläche} als die Oberflächenatome des Proteins, wobei d_{Oberfläche} ein Abstand vom Projektionszentrum zu einer maximalen z-Koordinate der Atome der Proteinoberfläche auf der z-Achse ist.

2. Verfahren zur Protein-Protein-Andockung auf Basis der Identifizierung einer Hydrierungsschicht mit geringer Entropie auf einer Proteinoberfläche gemäß Anspruch 1, wobei in Schritt 4 ein Verfahren zur Bezeichnung der verbleibenden Anpassungsebenen als die Oberflächenebenen und für jede Oberflächenebene das Auswählen des zentralen Atoms in der hydrophoben Verbindungsregion insbesondere umfasst:
Transformieren von Atomkoordinaten in einen dreidimensionalen Raum mit der Oberflächenebene als xy-Ebene durch Koordinatentransformation; Nehmen von xy-Koordinaten der Atomkoordinaten, wenn in einem Kreis, der auf Sauerstoff- und Stickstoffatomen zentriert ist und einen Radius von 5 Angström aufweist, keine anderen Sauerstoff- und Stickstoffatome vorhanden sind, Nichtbenutzen dieses Atoms als Grenze in einer nachfolgenden Suche nach der Grenze; andernfalls, in der xy-Ebene, mit Sauerstoff- und Stickstoffatomen als Grenzen, Zuweisen von Kohlenstoff- und Schwefelatomen als 1 in einem Kreis, der auf entsprechenden Sauerstoff- und Stickstoffatomen zentriert ist und einen Radius von 3 Angström aufweist; Nehmen von zugewiesenen Kohlenstoff- und Schwefelatomen als Zentrum, wenn es nicht zugewiesene Kohlenstoff- und Schwefelatome in einem Kreis gibt, der auf die zugewiesenen Kohlenstoff- und Schwefelatome zentriert ist und einen Radius von 3 Angström aufweist, Addieren von Werten der zugewiesenen Kohlenstoff- und Schwefelatomen in einem Kreis, der auf nicht zugewiesene Kohlenstoff- und Schwefelatome zentriert ist und einen Radius von 3 Angström aufweist, als Werte der nicht zugewiesenen Kohlenstoff- und Schwefelatome; zu diesem Zeitpunkt Berechnen von ausschließlich der Werte der nicht zugewiesenen Kohlenstoff- und Schwefelatome und Aufzeichnen als Pseudo-Bewertung der nicht zugewiesenen Kohlenstoff- und Schwefelatome und Nichtzuweisen der nicht zugewiesenen Kohlenstoff- und Schwefelatome; wenn alle Atome für eine Runde gesucht wurden, Zuweisen der Pseudo-Bewertung der nicht zugewiesenen Kohlenstoff- und Schwefelatome zu den entsprechenden nicht zugewiesenen Kohlenstoff- und Schwefelatomen und Starten einer neuen Runde der Zuweisung, bis alle Kohlenstoff- und Schwefelatome die Zuweisung abgeschlossen haben; und
für jedes Kohlenstoff- und Schwefelatom in einem auf die Kohlenstoff- und Schwefelatome zentrierten Kreis mit einem Radius von 3 Angström, wenn die Werte der Kohlenstoff- und Schwefelatome größer oder gleich den Werten der umgebenden Atome sind, Betrachten der Kohlenstoff- und Schwefelatome als zentrale Atome der entsprechenden hydrophoben Verbindungsregion.

3. Verfahren zur Protein-Protein-Andockung auf Basis der Identifizierung einer Hydrierungsschicht mit geringer Entropie auf einer Proteinoberfläche gemäß Anspruch 2, wobei in Schritt 4 ein Verfahren zum Markieren von Atomen in der hydrophoben Verbindungsregion insbesondere umfasst:
Nehmen des zentralen Atoms der hydrophoben Verbindungsregion als Zentrum und 10° als Schrittgröße, Teilen eines Bereichs um das zentrale Atom und Auswählen von Atomen mit einem Wert von größer oder gleich 3 in einem fächerförmigen Bereich, der jedem 10° entspricht; wenn ein Atom mit einem Wert von weniger als 3 zum ersten Mal auftritt, Auswählen eines Abstands von einem Atom mit einem Wert von 3, das dem Atom mit einem Wert von weniger als 3 am nächsten ist, zum Zentrum als Grenzabstand; und Auswählen von Atomen innerhalb des Grenzabstands als Atome innerhalb der hydrophoben Verbindungsregion.

4. Verfahren zur Protein-Protein-Andockung auf Basis der Identifizierung einer Hydrierungsschicht mit geringer Entropie auf einer Proteinoberfläche nach Anspruch 3, wobei in Schritt 5 ein Verfahren zur Berechnung der hydrophoben Fläche der hydrophoben Atome auf der Oberfläche jeder hydrophoben Verbindungsregion separat insbesondere umfasst:
Schritt 5.1, für die oberflächenschweren Atome des Proteins in jeder hydrophoben Verbindungsregion, Darstellen der oberflächenschweren Atome als eine Kugel mit einem Aktionsradius von 1,8 Angström; mit jedem oberflächenschweren Atom als Zentrum, Bilden einer Halbkugel in einer Projektionsrichtung, wobei die Halbkugel eine halbkugelförmige Schale ist;
Schritt 5.2, Erstellen eines zweidimensionalen Gitters mit einem Intervall von 0,1 Angström auf einer Ebene der hydrophoben Verbindungsregion und Aufzeichnen von Höheninformationen und eines Schweratomtyps der entsprechenden Proteinoberfläche in jedem zweidimensionalen Gitter; und
Schritt 5.3, Herstellen einer Oberfläche mit einem Radius von 1,8 Angström als Aktionsradius der Schweratome, mit der Möglichkeit von Hohlräumen und Diskontinuitäten auf der Oberfläche, wobei die halbkugelförmigen Schalen der beiden Schweratome keine Überschneidung haben und ein Abstand zwischen den beiden Atomen weniger als 6 Angström beträgt, wie in FIG. 3 gezeigt; Nehmen einer Verbindung zwischen einem fächerförmigen Bereich, der durch die halbkugelförmige Schale eines Schweratoms mit einer Projektionsrichtung in einem eingeschlossenen Winkel von 45° gebildet wird, und einem fächerförmigen Bereich, der durch die halbkugelförmige Schale eines anderen Schweratoms mit der Projektionsrichtung in einem eingeschlossenen Winkel von 45° gebildet wird, als Interpolationsbereich; Nehmen von Schnittpunkten der Verbindung und der zwei fächerförmigen Bereiche, die durch die halbkugelförmigen Schalen der zwei Schweratome mit der Projektionsrichtung in einem eingeschlossenen Winkel von 45° gebildet werden, als Interpolationsendpunkte, Erhalten einer Ebene eines Hohlraums zwischen den halbkugelförmigen Schalen der zwei Schweratome durch eine Interpolation, und Verkürzen der Ebene des Hohlraums als eine Interpolationsebene, wie in FIG. 4 gezeigt; und
am Hohlraum, Auswählen einer Oberfläche des Schweratoms unter 45° zur Projektionsrichtung als Oberflächenverbindungspunkt und Durchführen einer kubischen Spline-Interpolation, um einen Typ des Schweratoms und eine dreidimensionale Höhe der Interpolation am Hohlraum zu erhalten, und dann Bestimmen einer Raumfläche, die jedem Gitter in dem zweidimensionalen Gitter entspricht, und dann Bestimmen einer Fläche der hydrophoben Verbindungsregion.

5. Verfahren zur Protein-Protein-Andockung auf Basis der Identifizierung einer Hydrierungsschicht mit geringer Entropie auf einer Proteinoberfläche nach Anspruch 4, wobei in Schritt 6 ein Verfahren zur Auswahl der ersten drei hydrophoben Verbindungsregionen mit einer maximalen hydrophoben Fläche im Protein als mögliche Andockpositionen und das Andocken der zwei anzudockenden Proteine insbesondere umfasst:
Bezeichnen der hydrophoben Verbindungsregionen der beiden anzudockenden Proteine als eine hydrophobe Verbindungsregion A bzw. eine hydrophobe Verbindungsregion B; Bestimmen eines Suchbereichs auf der hydrophoben Verbindungsregion A oder der hydrophoben Verbindungsregion B gemäß einem relativ hohen durchschnittlichen Abstand d=max(di, dj) entsprechend den beiden hydrophoben Verbindungsregionen; Festlegen der hydrophoben Verbindungsregion A, Erstellen eines 2d × 2d zweidimensionalen Gitters mit einem Intervall von 3 Angström unter Verwendung einer Mittelkoordinate der hydrophoben Verbindungsregion A als Oberflächenursprung der hydrophoben Verbindungsregion, und Bezeichnen von Flächengrenzen des Suchbereichs entsprechend den 2d × 2d der hydrophoben Verbindungsregion A als (x_max, y_max); Lokalisieren einer Mittelkoordinate der hydrophoben Verbindungsregion B in Gitterpunkten des zweidimensionalen Gitters in Folge und Drehen in einem Intervall von 5° und Berechnen einer Andocksituation der zwei hydrophoben Verbindungsregionen an jedem Gitterpunkt; ein Berechnungsverfahren umfasst die folgenden Schritte:
Nehmen eines Normalenvektors einer Anpassungsebene sowohl der hydrophoben Verbindungsregion A als auch der hydrophoben Verbindungsregion B als z-Achse der Anpassungsebene, Annähern der beiden hydrophoben Verbindungsregionen auf der z-Achse der Anpassungsebene, Anordnen der hydrophoben Verbindungsregion B über der hydrophoben Verbindungsregion A und allmähliches Verringern einer Höhe der hydrophoben Verbindungsregion B, d. h. allmähliches Annähern der hydrophoben Verbindungsregion B an die hydrophobe Verbindungsregion A; wobei das allmähliche Annähern der beiden hydrophoben Verbindungsregionen auf der Ebene darin besteht, die z-Achsen der beiden hydrophoben Verbindungsregionen allmählich zu überlappen; wenn jedoch die z-Achse der hydrophoben Verbindungsregion A nach oben weist, weist die z-Achse der hydrophoben Verbindungsregion B nach unten, was der Durchführung einer Koordinatentransformation auf der hydrophoben Verbindungsregion B ähnlich ist; und nach der Transformation befinden sich die beiden Bereiche in einem gleichen Koordinatensystem;
gemäß dem Verfahren der allmählichen Annäherung der hydrophoben Verbindungsregion B an die hydrophobe Verbindungsregion A, Festlegen eines Mindestabstands von 1 Angström zwischen den Atomen auf der hydrophoben Verbindungsregion A und der hydrophoben Verbindungsregion B, und Bezeichnen einer Position des Mindestabstands als Raum-Andockposition; Ermitteln der minimalen x- und y-Achsenkoordinaten der Atomkoordinaten der hydrophoben Verbindungsregion A und der hydrophoben Verbindungsregion B in der räumlichen Andockposition, die als (x_min, y_min) bezeichnet werden, um die nachfolgende Gittereinrichtung und die Andockberechnung zu erleichtern;
nachdem die Koordinaten (x_min, y_min) erhalten wurden, Pressen der Atome in den hydrophoben Verbindungsregionen A und B in eine x-y-Ebene, Berechnen der Atome, die der Koordinate (x_min, y_min) in den hydrophoben Verbindungsregionen A bzw. B in der x-y-Ebene am nächsten sind, Bezeichnen von zwei erhaltenen Atomen als ein Atom a und ein Atom b, Nehmen eines Durchschnitts der z-Achsenhöhen, die den jeweiligen räumlichen Koordinaten entsprechen, die durch das Atom a und das Atom b unter der Raum-Andockposition repräsentiert werden, als einen z-Wert der Andockebene, und dann Bestimmen einer dreidimensionalen räumlichen Koordinate (x_min, y_min, z); Berechnen eines realen Abstands von dem Atom a und dem Atom b in der räumlichen Andockposition getrennt unter Verwendung der Koordinate (x_min, y_min, z), und wenn es einen Abstand von nicht weniger als 6 Angström in den zwei Abständen gibt, Bestimmen, dass die hydrophobe Verbindungsregion A und die hydrophobe Verbindungsregion B keine Andockfläche an der Koordinate haben; andernfalls Bestimmen, dass es eine Andockfläche zwischen zwei Proteinen an der Koordinate gibt;
wenn es an der aktuellen Raum-Andockposition keine Andockfläche gibt, Verschieben der hydrophoben Verbindungsregion B auf dem zweidimensionalen Gitter der hydrophoben Verbindungsregion A mit einer Bewegungsschrittgröße von (x, y) um 0,1 Angström, d. h.: y erhöht sich einmal um 0.1, und x geht von x_min nach x_max; und y erhöht sich noch einmal um 0,1, und x geht noch einmal von x_min nach x_max; Wiederholen des obigen Schritts, wobei sich der Bereich von y von y_min nach y_max ändert, bis alle Koordinaten durchlaufen sind;
wenn es eine Andockfläche zwischen den beiden Proteinen gibt, Aufzeichnen des Typs der Andockfläche unter der Raum-Andockposition;
wenn es eine Andockfläche zwischen den beiden Proteinen gibt, Bestimmen eines Andocktyps an einer Andockschnittstelle der hydrophoben Verbindungsregionen A und B unter der aktuellen Raum-Andockposition, und Einstellen des Wertes der Andockebene z auf eine z-Achsenhöhe, die einem gleichen Abstand von dem Atom a und dem Atom b entspricht; wobei
der Andocktyps Kohlenstoffatom-Kohlenstoffatom, Kohlenstoffatom-Sauerstoff- und Stickstoffatome und Sauerstoff- und Stickstoffatome-Sauerstoff- und Stickstoffatome umfasst, und Flächen verschiedener Andocktyps an der Andockschnittstelle der hydrophoben Verbindungsregionen A und B entsprechend dem Andocktyp berechnet werden.

6. Verfahren zur Protein-Protein-Andockung auf Basis der Identifizierung einer Hydrierungsschicht mit geringer Entropie auf einer Proteinoberfläche gemäß Anspruch 5, wobei in Schritt 6, wenn es mehrere Andockpositionen gibt, eine maximale vollständige Andockfläche als die Andockposition ausgewählt wird und eine vollständige Andockfläche der beiden Proteine an der Position integral berechnet wird.

7. Verfahren zur Protein-Protein-Andockung auf Basis der Identifizierung einer Hydrierungsschicht mit geringer Entropie auf einer Proteinoberfläche, umfassend einen Speicher, wobei der Speicher mindestens einen Befehl speichert und der mindestens eine Befehl von einem Prozessor geladen und ausgeführt wird, um das Verfahren zum Protein-Protein-Andockung auf Basis der Identifizierung einer Hydierungsschicht mit geringer Entropie auf einer Proteinoberfläche gemäß einem der Ansprüche 1 bis 6 zu implementieren.

8. Verfahren zur Protein-Protein-Andockung auf Basis der Identifizierung einer Hydrierungsschicht mit geringer Entropie auf einer Proteinoberfläche gemäß Anspruch 7, ferner umfassend einen Prozessor, wobei der Prozessor den mindestens einen im Speicher gespeicherten Befehl lädt und ausführt, um das Verfahren zur Protein-Protein-Andockung auf Basis der Identifizierung einer Hydrierungsschicht mit geringer Entropie auf einer Proteinoberfläche gemäß einem der Ansprüche 1 bis 6 zu implementieren.

## Revendications

1. Procédé pour l'accueil protéine-protéine sur la base de l'identification d'une couche d'hydratation à faible entropie sur la surface protéique, comprenant les étapes suivantes :
étape 1, frotter des atomes lourds sur une surface protéique, les atomes lourds comprenant des atomes de carbone, des atomes d'azote, des atomes d'oxygène et des atomes de soufre ; calculer une coordonnée spatiale moyenne des atomes lourds dans la protéine et diviser les atomes de protéine en 20 régions avec 20 sommets d'un dodécaèdre régulier et utiliser la coordonnée spatiale moyenne comme centre de projection ;
étape 2, pour la protéine dans chaque zone de frottement obtenue, utiliser une direction du centre de projection vers les sommets du dodécaèdre régulier comme axe z, et déterminer les atomes de surface de la protéine en fonction d'une distance maximale z entre le centre de projection et les atomes de protéine sur l'axe z ;
étape 3, parcourir les atomes de surface et identifier les atomes hydrophobes en fonction des acides aminés et des atomes de surface de la surface protéique, dans lequel
cas 1 : si les acides aminés de la surface protéique sont choisis dans le groupe constitué par la leucine (Leu), la tyrosine (Tyr), le tryptophane (Trp), l'isoleucine (Ile), la méthionine (Met), la phénylalanine (Phe), l'arginine (Arg) et la lysine (Lys), et lorsque les atomes d'oxygène carbonyle ou les atomes d'azote amide du squelette des acides aminés sont exposés à la surface protéique, identifier les atomes d'oxygène de carbonyle ou les atomes d'azote d'amide sur le squelette comme atomes hydrophobes, indiquer que des atomes d'oxygène et d'azote hydrophiles du squelette du résidu Leu, Tyr, Trp, Ile, Met, Phe, Arg, ou Lys deviennent les atomes hydrophobes ;
cas 2 : si les acides aminés de la surface protéique appartiennent aux résidus Trp, Tyr, Lys et Met, identifier les atomes d'oxygène et d'azote des chaînes latérales des résidus des acides aminés comme atomes hydrophobes, indiquer que les atomes d'oxygène et d'azote hydrophiles au niveau de la tête des chaînes latérales des résidus Trp, Tyr, Lys et Met deviennent des atomes hydrophobes ; et
cas 3 : si des liaisons hydrogène sont formées entre les atomes d'oxygène et d'azote de la surface protéique, que ce soit dans une « hélice a » et un « feuillet β » d'une structure secondaire de la protéine ou ailleurs, identifier les atomes d'oxygène et d'azote qui forment les liaisons hydrogène comme des atomes hydrophobes ;
étape 4, après avoir identifié les atomes hydrophobes dans les 20 régions de la protéine à l'étape 3, réajuster un plan, dans chacune des 20 régions, aux atomes de surface de la protéine par une méthode des moindres carrés, chaque plan ainsi obtenu étant utilisé comme plan d'accueil candidat de protéine ; calculer une distance moyenne di entre une position de coordonnées centrales (xi, yi) de chacun des plans d'accueil de protéine dans les 20 régions et tous les atomes sur le plan, dans lequel i est un numéro de série de la zone de frottement ;
après avoir ajusté 20 plans, comparer les plans obtenus et exclure les plans en double ; pendant l'exclusion des plans en double, si deux plans d'ajustement ont un angle inclus inférieur ou égal à 10° entre une origine spatiale et des vecteurs verticaux des plans respectifs, considérer les deux plans d'ajustement comme un même plan ; et
désigner les plans d'ajustement restants comme des plans de surface et, pour chaque plan de surface, sélectionner un atome central dans une région de connexion hydrophobe et marquer les atomes dans la région de connexion hydrophobe ;
étape 5, calculer une zone hydrophobe des atomes hydrophobes sur une surface de chaque région de connexion hydrophobe séparément ; et
étape 6, sélectionner les trois premières régions de connexion hydrophobes présentant une zone hydrophobe maximale dans la protéine en tant que positions d'accueil possibles, et accueillir les deux protéines à accueillir ;
dans lequel l'étape 1 comprend spécifiquement :
la lecture des informations de données contenues dans un fichier de structure de la banque de données de protéine (PDB) de la protéine, afin d'obtenir une coordonnée spatiale tridimensionnelle de chaque atome lourd sur la surface protéique ; et
la division des atomes de protéine en 20 régions avec les 20 sommets du dodécaèdre régulier comme suit : le dodécaèdre régulier a 20 sommets et la coordonnée spatiale moyenne de tous les atomes de la protéine est utilisée comme origine spatiale ; si, dans une zone spatiale, un angle est inférieur à 41° entre un vecteur pointant vers chaque atome depuis l'origine spatiale et un vecteur pointant vers le sommet, l'atome est divisé dans cette zone spatiale ; les 20 sommets du dodécaèdre régulier divisent les atomes de protéine en 20 zones, et une zone divisée de la surface protéique est considérée comme une zone de frottement ; et
dans lequel, à l'étape 2, un processus de détermination des atomes de surface protéique en fonction de la distance maximale z entre le centre de projection et les atomes de protéine sur l'axe z comprend spécifiquement :
la sélection des atomes latéraux externes sur 30 % de la distance maximale z entre le centre de projection et les atomes de protéine sur l'axe z en tant qu'atomes de surface de la protéine, c'est-à-dire la sélection des atomes dont la distance par rapport au centre de projection est supérieure à 70 % x d_{surface} en tant qu'atomes de surface de la protéine, dans lequel la d_{surface} est une distance entre le centre de projection et une coordonnée z maximale des atomes de la surface protéique sur l'axe z.

2. Procédé d'accueil protéine-protéine sur la base de l'identification d'une couche d'hydratation à faible entropie sur la surface protéique selon la revendication 1, dans lequel, à l'étape 4, un processus consistant à désigner les plans d'ajustement restants comme étant les plans de surface et, pour chaque plan de surface, à sélectionner l'atome central dans la région de connexion hydrophobe comprend spécifiquement :
la transformation des coordonnées atomiques en un espace tridimensionnel dont le plan de surface est un plan xy par transformation de coordonnées ; la prise des coordonnées xy des coordonnées atomiques, si dans un cercle centré sur les atomes d'oxygène et d'azote et ayant un rayon de 5 angströms, il n'y a pas d'autres atomes d'oxygène et d'azote, la non-utilisation de cet atome comme limite dans une recherche ultérieure de la limite ; sinon, dans le plan xy, avec les atomes d'oxygène et d'azote comme limites, l'assignation aux atomes de carbone et de soufre la valeur 1 dans un cercle centré sur les atomes d'oxygène et d'azote correspondants et ayant un rayon de 3 angströms ; la prise des atomes de carbone et de soufre assignés comme centre, s'il y a des atomes de carbone et de soufre non assignés dans un cercle centré sur les atomes de carbone et de soufre assignés et avec un rayon de 3 angströms, l'addition des valeurs des atomes assignés dans un cercle centré sur les atomes de carbone et de soufre non assignés et avec un rayon de 3 angströms comme valeurs des atomes de carbone et de soufre non assignés ; à ce moment-là, le calcul uniquement des valeurs des atomes de carbone et de soufre non assignés, et l'enregistrement comme pseudo-évaluation des atomes de carbone et de soufre non assignés, tout en n'assignant pas les atomes de carbone et de soufre non assignés ; lorsque tous les atomes ont été recherchés pendant un tour, l'assignation de la pseudo-évaluation des atomes de carbone et de soufre non assignés aux atomes de carbone et de soufre non assignés correspondants, et le commencement d'un nouveau tour d'assignation jusqu'à ce que tous les atomes de carbone et de soufre aient achevé l'assignation ; et
pour chaque atome de carbone et de soufre, dans un cercle centré sur les atomes de carbone et de soufre et d'un rayon de 3 angströms, si les valeurs des atomes de carbone et de soufre sont supérieures ou égales aux valeurs des atomes environnants, considérer les atomes de carbone et de soufre comme des atomes centraux de la région de connexion hydrophobe correspondante.

3. Procédé d'accueil protéine-protéine sur la base de l'identification d'une couche d'hydratation à faible entropie sur la surface protéique selon la revendication 2, dans lequel, à l'étape 4, un processus de marquage des atomes dans la région de connexion hydrophobe comprend spécifiquement :
la prise de l'atome central de la région de connexion hydrophobe comme centre et 10° comme taille de pas, la division d'une zone autour de l'atome central et la sélection des atomes ayant une valeur supérieure ou égale à 3 dans une zone en forme d'éventail correspondant à chaque 10° ; lorsqu'un atome ayant une valeur inférieure à 3 apparaît pour la première fois, la sélection d'une distance entre l'atome ayant une valeur de 3 le plus proche de l'atome ayant une valeur inférieure à 3 et le centre comme distance de coupure ; et la sélection des atomes situés à l'intérieur de la distance de coupure comme atomes à l'intérieur de la région de connexion hydrophobe.

4. Procédé d'accueil protéine-protéine sur la base de l'identification d'une couche d'hydratation à faible entropie sur la surface protéique selon la revendication 3, dans lequel à l'étape 5, un processus de calcul de la zone hydrophobe des atomes hydrophobes sur la surface de chaque région de connexion hydrophobe séparément comprend spécifiquement :
étape 5.1, pour les atomes lourds de surface protéique dans chaque région de connexion hydrophobe, l'affichage des atomes lourds de surface sous forme de sphère avec un rayon d'action de 1,8 angström ; avec chaque atome lourd de surface comme centre, la formation d'un hémisphère dans une direction de projection, dans lequel l'hémisphère est une coque hémisphérique ;
étape 5.2, l'établissement d'une grille bidimensionnelle avec un intervalle de 0,1 angström sur un plan de la région de connexion hydrophobe, et l'enregistrement des informations sur la hauteur et un type d'atome lourd de la surface protéique correspondante dans chaque grille bidimensionnelle ; et
étape 5.3, l'établissement d'une surface ayant un rayon de 1,8 angström comme rayon d'action des atomes lourds, avec possibilité de vides et de discontinuités sur la surface, dans lequel les coques hémisphériques des deux atomes lourds n'ont pas d'intersection, et une distance entre les deux atomes est inférieure à 6 angströms, comme le montre la FIG. 3 ; la prise d'une connexion entre une région en forme d'éventail formée par la coque hémisphérique d'un atome lourd avec une direction de projection dans un angle inclus de 45° et une région en forme d'éventail formée par la coque hémisphérique d'un autre atome lourd avec la direction de projection dans un angle inclus de 45° comme une zone d'interpolation ; la prise des intersections de la connexion et des deux régions en forme d'éventail formées par les coques hémisphériques des deux atomes lourds avec la direction de projection dans un angle inclus de 45° comme points d'extrémité d'interpolation, l'obtention d'un plan d'une cavité entre les coques hémisphériques des deux atomes lourds par une interpolation, et l'abréviation du plan de la cavité comme un plan d'interpolation, comme illustré dans la FIG. 4 ; et
dans la cavité, la sélection d'une surface de l'atome lourd à 45° de la direction de projection comme point de connexion de surface, et la réalisation d'une interpolation de fonction spline cubique pour obtenir un type d'atome lourd et une hauteur tridimensionnelle de l'interpolation dans la cavité, puis la détermination d'une zone d'espace correspondant à chaque grille dans la grille bidimensionnelle, puis la détermination d'une zone de la région de connexion hydrophobe.

5. Procédé d'accueil protéine-protéine sur la base de l'identification d'une couche d'hydratation à faible entropie sur la surface protéique selon la revendication 4, dans lequel, à l'étape 6, un processus de sélection des trois premières régions de connexion hydrophobes avec une zone hydrophobe maximale dans la protéine en tant que positions d'accueil possibles, et l'accueil des deux protéines à accueillir comprend spécifiquement :
la désignation des régions de connexion hydrophobe des deux protéines à accueillir comme une région de connexion hydrophobe A et une région de connexion hydrophobe B, respectivement ; la détermination d'une plage de recherche sur la région de connexion hydrophobe A ou la région de connexion hydrophobe B en fonction d'une distance moyenne relativement élevée d=max(di, dj) correspondant aux deux régions de connexion hydrophobe ;
la fixation de la région de connexion hydrophobe A, l'établissement d'une grille bidimensionnelle 2d × 2d avec un intervalle de 3 angströms en utilisant une coordonnée centrale de la région de connexion hydrophobe A comme origine de surface de la région de connexion hydrophobe, et la désignation des limites de la zone de recherche correspondant à 2d × 2d de la région de connexion hydrophobe A comme (x_max, y_max) ; la localisation d'une coordonnée centrale de la région de connexion hydrophobe B dans des points de grille de la grille bidimensionnelle en séquence, et la rotation à un intervalle de 5° et le calcul d'une situation d'accueil des deux régions de connexion hydrophobe à chaque point de grille ; un procédé de calcul comprend les étapes suivantes :
la prise d'un vecteur normal d'un plan d'ajustement de chacune des régions de connexion hydrophobe A et de la région de connexion hydrophobe B comme axe z du plan d'ajustement, faire en sorte que les deux régions de connexion hydrophobe se rapprochent sur l'axe z du plan d'ajustement, le placement de la région de connexion hydrophobe B au-dessus de la région de connexion hydrophobe A, et la réduction progressive de la hauteur de la région de connexion hydrophobe B, c'est-à-dire faire en sorte que la région de connexion hydrophobe B se rapproche progressivement de la région de connexion hydrophobe A ; le rapprochement progressif des deux régions de connexion hydrophobe sur le plan consistant à faire se chevaucher progressivement les axes z des deux régions de connexion hydrophobe ; toutefois, si l'axe z de la région de connexion hydrophobe A est orienté vers le haut, l'axe z de la région de connexion hydrophobe B est orienté vers le bas, ce qui revient à effectuer une transformation de coordonnées sur la région de connexion hydrophobe B ; après la transformation, les deux régions se trouvent dans le même système de coordonnées ;
selon le processus de rapprochement progressif de la région de connexion hydrophobe B de la région de connexion hydrophobe A, la fixation d'une distance minimale de 1 angström entre les atomes de la région de connexion hydrophobe A et de la région de connexion hydrophobe B, et la désignation d'une position de la distance minimale comme position d'accueil spatiale ; dans la position d'accueil spatiale, la découverte des coordonnées minimales des axes x et y des coordonnées atomiques de la région de connexion hydrophobe A et de la région de connexion hydrophobe B, désignées par (x_min, y_min), de manière à faciliter l'établissement ultérieur de la grille et le calcul de l'accueil ;
une fois les coordonnées (x_min, y_min) obtenues, le pressage des atomes dans les régions de connexion hydrophobe A et B sur un plan x-y, le calcul des atomes les plus proches des coordonnées (x_min, y_min) dans les régions de connexion hydrophobe A et B dans le plan x-y, respectivement, la désignation des deux atomes obtenus comme un atome a et un atome b, la prise d'une moyenne des hauteurs de l'axe z correspondant aux coordonnées spatiales respectives représentées par l'atome a et l'atome b dans la position d'accueil spatiale comme valeur z du plan d'accueil, puis la détermination d'une coordonnée spatiale tridimensionnelle (x_min, y_min, z) ; le calcul d'une distance réelle entre l'atome a et l'atome b dans la position d'accueil spatiale séparément en utilisant la coordonnée (x_min, y_min, z) et, s'il existe une distance d'au moins 6 angströms entre les deux distances, la détermination que la région de connexion hydrophobe A et la région de connexion hydrophobe B n'ont pas de surface d'accueil au niveau de la coordonnée ; sinon, la détermination qu'il existe une surface d'accueil entre les deux protéines au niveau de la coordonnée ;
s'il n'y a pas de surface d'accueil au niveau de la position d'accueil spatiale actuelle, le déplacement de la région de connexion hydrophobe B sur la grille bidimensionnelle de la région de connexion hydrophobe A, avec un pas de déplacement de (x, y) de 0,1 angström, c'est-à-dire : y augmente de 0,1 une fois, et x passe de x_min à x_max ; et y augmente à nouveau de 0,1 fois, et x passe une nouvelle fois de x_min à x_max ; la répétition de l'étape ci-dessus, avec une plage de y passant de y_min à y_max, jusqu'à ce que toutes les coordonnées aient été parcourues ;
lorsqu'il existe une surface d'accueil entre les deux protéines, l'enregistrement d'un type de surface d'accueil sous la position d'accueil spatiale ;
lorsqu'il existe une surface d'accueil entre les deux protéines, la détermination d'un type d'accueil au niveau d'une interface d'accueil des régions de connexion hydrophobe A et B dans la position d'accueil spatiale actuelle, et l'ajustement de la valeur z du plan d'accueil au niveau d'une hauteur de l'axe z correspondant à une même distance de l'atome a et de l'atome b ; dans lequel
le type d'accueil comprend les atomes de carbone-carbone, les atomes de carbone-oxygène et d'azote, et les atomes d'oxygène et d'azote-oxygène et azote, et les zones des différents types d'accueil au niveau de l'interface d'accueil des régions de connexion hydrophobes A et B sont calculées en fonction du type d'accueil.

6. Procédé d'accueil protéine-protéine sur la base de l'identification d'une couche d'hydratation à faible entropie sur la surface protéique selon la revendication 5, dans lequel, à l'étape 6, s'il existe plusieurs positions d'accueil, une zone d'accueil complète maximale est sélectionnée comme position d'accueil, et une zone d'accueil complète des deux protéines est calculée intégralement au niveau de la position.

7. Dispositif d'accueil protéine-protéine sur la base de l'identification d'une couche d'hydratation à faible entropie sur la surface protéique, comprenant une mémoire, dans lequel la mémoire stocke au moins une instruction, et l'au moins une instruction est chargée et exécutée par un processeur pour mettre en oeuvre le procédé d'accueil protéine-protéine sur la base de l'identification d'une couche d'hydratation à faible entropie sur la surface protéique selon l'une quelconque des revendications 1 à 6.

8. Dispositif d'accueil protéine-protéine sur la base de l'identification d'une couche d'hydratation à faible entropie sur la surface protéique selon la revendication 7, comprend en outre un processeur, dans lequel le processeur charge et exécute l'au moins une instruction stockée dans la mémoire pour mettre en oeuvre le procédé d'accueil protéine-protéine sur la base de l'identification d'une couche d'hydratation à faible entropie sur une surface protéique selon l'une quelconque des revendications 1 à 6.
